Europäisches Patentamt

(19) European Patent Office

Office européen des brevets

(11) Publication number: **0 028 033**
**B1**

(12) EUROPEAN PATENT SPECIFICATION

(45) Date of publication of patent specification: **19.02.86**

(21) Application number: **80106685.3**

(22) Date of filing: **30.10.80**

(51) Int. Cl.⁴: **C 12 N 15/00,** C 12 P 21/02, C 12 N 1/20, C 07 H 21/04 // C12R1/19

(54) **Double-stranded DNA which codes for a polypeptide with human fibroblast interferon activity, cloned DNA, recombinant plasmid containing the DNA and microorganism containing the recombinant plasmid.**

(30) Priority: **30.10.79 JP 139289/79**
**19.03.80 JP 33931/80**

(43) Date of publication of application:
**06.05.81 Bulletin 81/18**

(45) Publication of the grant of the patent:
**19.02.86 Bulletin 86/08**

(84) Designated Contracting States:
**AT BE CH DE FR GB IT LI NL SE**

(56) References cited:

RESEARCH DISCLOSURE no. 183, July 1979, ref:18.309. pages 361-362. "The production of interferon by genetic engineering"

PROC. JAPAN. ACAD. 55 (9) Ser. B (1979), pages 464-469 T. TANIGUCHI et al. "Construction and identification of a bacterial plasmid containing the human fibroblast interferon gene sequence"

GENE, vol. 10, May 1980, pages 11-15. T. TANIGUCHI et al. "The nucleotide sequence of human fibroblast interferon cDNA"

(73) Proprietor: **Juridical Foundation Japanese Foundation for Cancer Research**
**37-1, Kami-Ikebukuro 1-chome Toshima-ku Tokyo (JP)**

(72) Inventor: **Sugano, Haruo**
**4-8-13, Minami-Ogikubo Suginami-ku Tokyo (JP)**
Inventor: **Muramatsu, Masami**
**4-21-6, Kotesashi-cho Tokorozawa-shi Saitama-ken (JP)**
Inventor: **Taniguchi, Tadatsugu**
**4-27-12-303, Tagara Nerima-ku Tokyo (JP)**

(74) Representative: **Vossius Vossius Tauchner Heunemann Rauh**
**Siebertstrasse 4 P.O. Box 86 07 67 D-8000 München 86 (DE)**

(56) References cited:
PROC. NATL. ACAD. SCI USA, vol. 77, (7), July 1980, pages 4003-4006. T. TANIGUCHI et al. "Molecular cloning of human interferon cDNA

## 0 028 033

**Description**

Interferon is a glycoprotein (molecular weight approx. 20,000) with antiviral activity, discovered by Isaacs and Lindenmann in 1957. Subsequent studies have indicated antitumor activity of the substance in addition to antiviral activity and hence a wide clinical application of this substance is expected. For instance, it has been reported that interferon may be effectively used to treat various viral diseases, osteosarcoma and mammary carcinoma.

However, because of its high species-specificity, only the interferon derived from human cells can be used for human application. At present, the interferon which is being used for administration has a relative activity of about $10^6$ (International units) per 1 mg, which corresponds to a purity of about 0.1—0.01%.

Moreover, the use of the interferon is quite limited because of difficulties in mass production. At present only about 1% of the interferon requirement even for clinical tests ($10^{13}$ units per annum) can be met. For these reasons there is a great need to develop technology to produce human interferon in high purity, with ease and in large quantities.

According to the present invention a double-stranded DNA is provided which codes for a polypeptide with human fibroblast interferon activity and which is complementary to a human fibroblast interferon mRNA which directs the synthesis of biologically active human fibroblast interferon in the Xenopus laevis oocyte translation system. Furthermore, there is provided a cloned DNA, a recombinant plasmid containing the DNA and a microorganism harbouring the recombinant plasmid.

In accordance with the present invention, a double-stranded DNA which codes for a polypeptide with human fibroblast interferon activity is prepared using the human interferon messenger RNA as a template and a novel recombinant plasmid containing the DNA is prepared. In addition, the recombinant plasmid may be inserted into a host microorganism.

The DNA which codes for a polypeptide with human fibroblast interferon activity and the recombinant plasmid containing the DNA have been obtained for the first time by the present inventors. The DNA and the recombinant plasmid may be used, *inter alia,* for amplification of human interferon in bacteria such as *Escherichia coli.*

The DNA and the recombinant plasmid of the present invention are prepared by the following general procedure.

First, cytoplasmic RNA is extracted from (1) human fibroblast, e.g. MG63 cells or others induced by poly(I): poly(C) (a double stranded RNA composed of polyinosinic acid and polycytidylic acid) or other inducers. From this RNA, the messenger RNA (hereinafter messenger RNA is referred to as mRNA) including human fibroblast interferon mRNA containing poly A (polyadenylic acid) is isolated. A double stranded DNA is synthesized, for example, by reverse transcriptase, with the mRNA preparation having high interferon mRNA activity as a template. A recombinant plasmid is obtained by inserting the synthesized DNA into a vector DNA such as *Escherichia coli* plasmid DNA by the technique of *in vitro* DNA recombination. The recombinant plasmid DNA is labelled with a radio isotope for use as a probe.

Recombinant plasmids having an inserted portion which is complementary to the human fibroblast interferon mRNA are selected. The DNA which codes for a polypeptide with human fibroblast interferon activity is recovered from the recombinant plasmid and the base sequence of the DNA is determined.

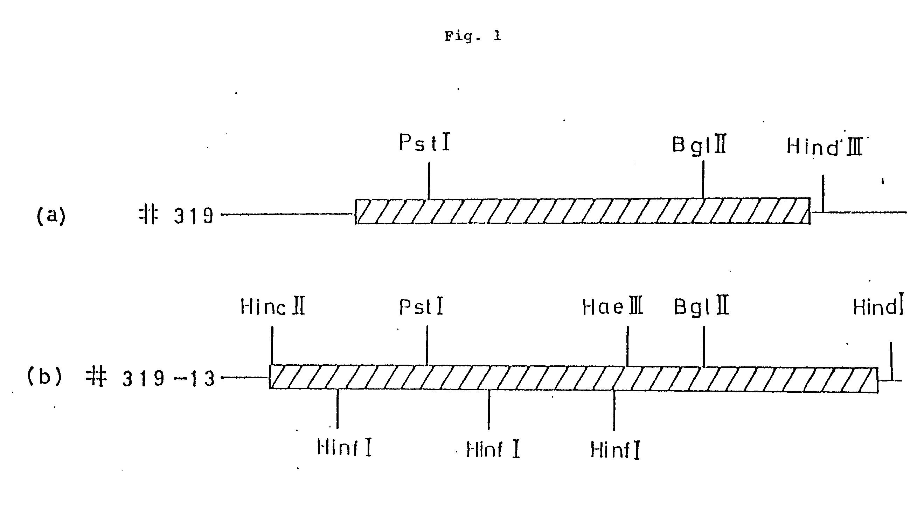

Fig. 1 illustrates restriction endonuclease maps of:

(a) a DNA segment in the recombinant plasmid No. 319 showing complementarity to the human fibroblast interferon mRNA which was used to select a novel recombinant plasmid No. 319-13; and

(b) a DNA segment in the novel recombinant plasmid No. 319-13 which shows complementarity to the human fibroblast interferon mRNA.

The DNA of the present invention may be a cloned DNA showing complementarity to the human fibroblast interferon mRNA, a cloned DNA which codes for a polypeptide with human fibroblast interferon activity or a cloned DNA which codes for human fibroblast interferon polypeptide. Especially a DNA which encompasses the entire coding region of the human fibroblast interferon mRNA is a preferred example of the DNA of the present invention.

In the recombinant plasmid of the present invention the DNA mentioned above is inserted in a vector DNA such as pBR322, pCR1, pMB9 or pSC1.

The recombinant plasmids named No. 319 and No. 319-13 are preferred examples of a recombinant plasmid according to the invention.

The recombinant plasmid and thus the DNA mentioned above are inserted in a host microorganism and the resulting transformant can be used to produce a substance having human fibroblast interferon activity.

As the host microorganism, *Escherichia coli* X1776 is preferably used.

An example of the processes of producing the DNA, the recombinant plasmid and the transformant of the present invention is as follows.

First, human fibroblasts may be obtained from fetus-derived foreskin, or the like. A small amount of interferon is then added to a culture fluid of human fibroblasts to prime the interferon synthesis by human fibroblasts, to which poly(I): poly(C) is added to induce the synthesis of interferon mRNA. Cycloheximide is added simultaneously to increase the level of interferon mRNA. At an appropriate time (about 4 hours) after the human fibroblasts have been superinduced in the above manner, cells are collected and destroyed and

2

the nuclei are removed. Cytoplasmic total RNA is extracted with phenol, or the like. The RNA can also be extracted by destroying the whole cells, extracting both DNA and RNA with, for example, phenol, and degrading and removing the DNA with DNAase.

Further, interferon mRNA containing cytoplasmic RNA can also be extracted from MG63 cells induced by poly(I): poly(C) or other inducers.

The thus extracted RNA is dissolved in a salt solution of NaCl or KCl at a high concentration such as 0.5 M and put on a column of oligo (dT) cellulose to adsorb polyadenylated mRNA on the column. Elution is carried out with water, a salt solution at a low concentration such as 10 mM Tris-HCl buffer, or the like to isolate poly(A) mRNA.

The isolated mRNA is fractionated by sucrose density gradient centrifugation. Interferon mRNA activity in each fraction is checked by determining interferon activity (antiviral activity) of the proteins which are synthesized in oocytes of African claw toad (*Xenopus laevis*) after microinjecting a part of the mRNA in each fraction. The determination of interferon activity is carried out according to the method described in Japan J. Microbiol. *18*, 449—456, (1974).

Then, a DNA showing complementarity to the mRNA is synthesized *in vitro* by a reverse transcriptase, which is obtained from avian myeloblastosis virus, using, as the template, an mRNA-fraction having the highest interferon mRNA activity.

The synthesis is carried out as follows:

An mRNA is reacted at an appropriate temperature (e.g. 37°C) for an appropriate period (e.g. 60 min.) with oligo(dT) as primer, $MgCl_2$ (e.g. 5 mM), NaCl (e.g. 30 mM), mercaptoethanol (e.g. 5 mM) and Tris-HCl buffer (e.g. pH 8.0, 40 mM) using a reverse transcriptase together with deoxyadenosine triphosphate (dATP), deoxythymidine triphosphate (dTTP), deoxyguanosine triphosphate (dGTP) and deoxycytidine triphosphate (dCTP) (e.g. 0.5 mM each) as substrates.

The thus obtained product—namely the mRNA/cDNA-hybrid—is subjected to deproteinization with, for example, phenol, and the template RNA is removed by alkali or ribonuclease H treatment. Using the remaining cDNA single strand the second strand is synthesized by a reverse transcriptase in a similar way as the synthesis of the DNA showing complementarity to mRNA described above except that mRNA is replaced by DNA and oligo(dT) is omitted.

By using *Escherichia coli* DNA polymerase I which can be obtained from *Escherichia coli* MRE 600, or the like, instead of reverse transcriptase, the same double stranded DNA can be synthesized.

The double stranded cDNA is treated with Nuclease $S_1$ (which can be obtained from *Aspergillus oryzae*) in the presence of $ZnCl_2$ (e.g. 1 mM), sodium acetate buffer (e.g. 0.1 M, pH 4.5), NaCl (e.g. 0.2 M), etc. Deoxyadenine chains are formed at both 3' ends of the double-stranded cDNA by incubating with a terminal transferase (purified from calf thymus) in the presence of potassium cacodylate buffer (e.g. pH 7.6, 0.14 M), Tris (base) (e.g. 0.03 M), dithiothreitol (e.g. 0.1 mM), $CoCl_2$ (e.g. 1 mM) and dATP (e.g. 1 mM) at an appropriate temperature (e.g. 37°C) for an appropriate period (e.g. 20 min.)

On the other hand, a plasmid DNA which is used as a vector DNA, e.g. *Escherichia coli* plasmid pBR322 DNA [Gene vol. 2, p. 95—113 (1977)], is cleaved at one site by treating with a restriction endonuclease EcoRI (which can be obtained, for example, from *Escherichia coli* RY13) in the presence of Tris HCl buffer (e.g. pH 7.5, 10 mM), $MgCl_2$ (e.g. 6 mM), NaCl (e.g. 0.1 M), mercaptoethanol (e.g. 6 mM), or the like and then treated with phage λ-derived exonuclease (which can be obtained, for example, from *Escherichia coli* W3102 (λ cl851)×13)) in the presence of Na-glycine buffer (e.g. pH 9.5, 0.1 M), $MgCl_2$ (e.g. 5 mM), or the like. Thereafter deoxythymidine chains are formed at both 3' ends in the same way as the deoxyadenine chains at the above-described double stranded cDNA by using dTTP instead of dATP.

Synthetic double stranded DNA and plasmid DNA which are chain-elongated at both 3' ends as described above are incubated at an appropriate temperature for an appropriate period with Tris-HCl buffer (e.g. pH 7.5, 50 mM), NaCl (e.g. 0.1 M), EDTA (e.g. 5 mM), or the like and hybridized by forming hydrogen bonds between the adenine- and thymidine-chains. Then, a transformable *Escherichia coli* strain, e.g. *Escherichia coli* χ1776 (Molecular Cloning of Recombinant DNA, Scott, W. A. & Werner, R. Edit., Academic Press p. 99—114, 1977) is transformed with the hybridized DNA according to the method of Enea et al. (J. Mol. Biol. vol. 96, p. 495—509, 1975) or the like.

In the novel recombinant plasmid DNA thus obtained, there exists a vector DNA gene, e.g. β-lactamase (enzyme that destroys ampicillin) gene, of *Escherichia coli* plasmid pBR322. Therefore, the transformed *Escherichia coli* shows resistance to ampicillin. The following technique is used to select among these ampillicin resistant strains a strain with a novel recombinant plasmid having a gene which shows complementarity to the human fibroblast interferon messenger RNA.

First, ($^{32}$P) labelled single stranded DNA is synthesized using the RNA having interferon mRNA activity described above as a templated. This DNA is hybridized with mRNA extracted from the human fibroblasts which were not induced by poly(I):poly(C) (therefore, interferon mRNA synthesis is not induced).

Hybridization occurs by incubating at a high temperature (e.g. 65°C) in a reaction mixture containing, for example NaCl (e.g. 0.5 M). Then, the DNA/mRNA-hybrid (Probe A) and non-hybridized DNA (Probe B) are separated by hydroxyapatite column chromatography. Next, filter-fixed DNAs of transformant colonies are hybridized separately with Probe B or Probe A according to the technique of Grunstein-Hogness (Proc. Nat. Acad. Sci. USA, vol. 72, p. 3961—3965, 1975) and colonies having a DNA hybridizable with Probe B but not or barely with Probe A are discovered by autoradiography.

3

Strains giving this response are propagated and their plasmid DNA is isolated. mRNA having interferon mRNA activity in the oocyte assay was prepared according to step I (Appendix II) and hybridized against the plasmid DNA by incubating at a high temperature (e.g. 53°C) in the presence of 80% (w/v) formamide, 0.4 M NaCl, etc. The mRNA hybridized with the cDNA portion of the plasmid DNA from the above described strain. Therefore, it can be retained on a nitrocellulose filter, whereas unhybridized mRNA can not under certain conditions (refer to Example below and Nygaard, A.P. & Hall, B.D., Biochem. Biophys. Res. Commun. Vol. 12, p. 98—104, 1963). This hybridized mRNA can be recovered selectively from the filter at a high temperature (e.g. 60°C) in a solution such as 90% (v/v) formamide. Thereafter it is injected into oocytes of Xenopus laevis.

When interferon is synthesized in the oocytes, the recombinant plasmid DNA used for hybridization must contain a DNA which is complementary to interferon mRNA; and by this method, a recombinant plasmid DNA having at least parts of a DNA sequence showing complementarity to the human fibroblast interferon mRNA can be isolated.

Hybridization with interferon mRNA can also be achieved with recombinant plasmid DNA in which only part of the interferon gene is inserted. Therefore, it is desirable to identify among the ampicillin resistant transformants obtained as described above strains containing recombinant plasmids the inserted DNA of which comprises possibly the entire information for human fibroblast interferon. Therefore, the cDNA of the recombinant plasmid obtained above and shown to hybridize with interferon mRNA or segments of it are cleaved with a restriction endonuclease. The fragments obtained are labelled with a radio isotope such as $^{32}$P by nick translation method (Rigby, et al., J. Mol. Biol. vol. 113, p. 237—251, 1977), or the like, and used as a probe. With this probe colony hybridization is performed as described above (technique of Grunstein and Hogness). Several strains thus obtained are cultured and the plasmid DNA is isolated therefrom. The plasmid DNA is cleaved with a restriction endonuclease to obtain the inserted DNA. The length of the inserted DNA is investigated to obtain a plasmid having an inserted DNA coding the entire region of the interferon protein. Primary structure of the inserted DNA of one of recombinant plasmids isolated by the above method is determined according to the Maxam-Gilbert method (Proc. Nat. Acad. Sci. U.S.A. vol. 74, p. 560—564, 1977) and is illustrated in the following Example. It has thus been shown that the recombinant plasmid of the invention contains the entire coding region corresponding to the human fibroblast interferon mRNA.

As outlined above, a DNA which codes for human fibroblast interferon polypeptide, especially a DNA which encompasses the entire coding region corresponding to the human fibroblast interferon mRNA, a recombinant plasmid containing the DNA and a microorganism containing the plasmid are prepared.

The base sequence of the DNA obtained above and the corresponding peptide sequence are illustrated in Table 5 below.

The base sequence in Table 5 is a preferred example for a DNA which codes for a polypeptide with human fibroblast interferon activity. Since the amino acids in the peptide sequence in Table 5 may be coded for by a base triplet other than those in Table 5, base sequences of the DNA which codes for a polypeptide with human fibroblast interferon activity other than that in Table 5 are also included in the present invention.

The present novel recombinant plasmids having a gene which encompasses at least the entire coding region of the human fibroblast interferon mRNA are useful for constructing strains which enable mass production of interferon in Escherichia coli or in eukaryotic cells which can be grown on a large scale.

Recombinant plasmids containing a DNA using, as a template, leucocyte mRNA or immune interferon mRNA can be prepared by the same method as mentioned above and such plasmids are also expected to be useful for the mass production of interferon.

One specific embodiment of the present invention is illustrated by the following representative example.

Example

(I) Preparation of double-stranded cDNA (dsDNA) of human fibroblast interferon.

After priming of human fibroblasts by overnight incubation with MEM culture medium (product of Nissui Seiyaku Co., Ltd., Japan) containing human interferon which is prepared according to the method described in Proc. Nat. Acad. Sci. USA, 73, 520—523 (1976) (25 U/ml), the fibroblasts were superinduced by adding 10 µg/ml of poly(I):poly(C) (product of Calbiochem Co., USA) and 5 µg/ml of cycloheximide to the medium. The priming and superinduction are carried out according to the methods described in Brit. J. Exp. Path., 39, 452—458 (1958) and Antimicrob. Agents Chemother., 2, 476—484 (1972), respectively.

After 4 hours, $1.5 \times 10^9$ superinduced human fibroblasts were destroyed by Teflon homogenizer (sold by Takashima Shoten Co., Japan) at a temperature of 0 to 4°C in the presence of 0.3% NP-40 (product of Daiichi Kagaku Co., Japan) and 50 µg/ml heparin in RSB buffer (10 mM Tris-HCl, pH 7.5; 10 mM NaCl; 1.5 mM $MgCl_2$). Nuclei were removed by centrifugation at 1,500×g and 4°C for 10 minutes and 9.6 mg of cytoplasmic RNA was obtained by extraction 3 times with phenol.

The cytoplasmic RNA was precipitated with 67% ethanol in the presence of 0.1 M NaCl, dissolved in 10 ml of 1 mM EDTA solution and incubated at 65°C for 2 minutes. Then, 2.5 ml of a salt solution at a high concentration (0.5 M Tris-HCl, pH 7.5; 1 M NaCl; 50 mM EDTA) was added to the above solution and the mixture was put on a column packed with 0.15 g of an oligo(dT) cellulose (product of P-L Biochemicals Co.,

USA) to adsorb polyadenylated mRNA. Elution was then carried out with a salt solution at a low concentration (10 mM Tris-HCl, pH 7.5) and water to isolate 250 µg of polyadenylated mRNA.

The mRNA was precipitated with 67% ethanol in the presence of 0.1 M NaCl and dissolved in 0.5 ml of 1 mM EDTA solution. The solution was incubated at 65°C for 2 minutes, subjected to centrifugation through a 5—25% sucrose-density gradient containing 50 mM Tris-HCl, pH 7.5, 0.2 M NaCl and 1 mM EDTA (rotated at 274,000×g using the SW40 rotor of Beckmann Co., U.S.A.) at 4°C for 16 hrs. and fractionated into 20 fractions.

The interferon mRNA activity of each of these fractions was determined as mentioned above, and the results are shown in Table 1 below.

### TABLE 1

| Fraction No. | Interferon activity |
| --- | --- |
| 9 | <50 units/ml |
| 10 | 44 |
| 11 | 550 |
| 12 | 52 |

The mRNA mixture in fraction no. 11 was approximately 5 µg. This mRNA and a reverse transcriptase were incubated at 37°C for an hour in 20 µl of a reaction mixture consisting of 5 µg mRNA; 0.5 mM dATP; 0.5 mM dTTP; 0.5 mM dGTP; 0.5 mM dCTP; 1 µg oligo(dT) (product of P-L Biochemicals Co., USA); 8 units reverse transcriptase (derived from Avian myeloblastosis virus, for example, product of Life Science Inc. Florida, USA); 5 mM $MgCl_2$; 30 mM NaCl; 5 mM mercaptoethanol; and 40 mM Tris-HCl (pH 8.0) and then deproteinized with phenol. The RNA strand was destroyed by treatment with 0.3 N NaOH at 37°C for 15 hours, and the synthesized single stranded DNA was incubated at 37°C in 20 µl of a reaction mixture [the same mixture as described above except that mRNA and oligo(dT) were omitted] for one hour to synthesize 1.5 µg of a double stranded DNA.

The double stranded DNA was treated with Nuclease $S_1$ (product of Bethesda Research Laboratories Inc., USA which is referred to as BRL, hereinafter) in 50 µl of a reaction mixture (1.5 µg double stranded DNA: 1 mM $ZnCl_2$; 0.1 M sodium acetate, pH 4.5; 0.2 M NaCl; 0.05 units $S_1$) at 37°C for 30 minutes and the enzyme was removed by phenol extraction. The resulting blunt ended DNA was precipitated with ethanol and then treated with a terminal transferase in 20 µl of a reaction mixture consisting of 1.5 µg duplex DNA; 0.14 M potassium cacodylate, pH 7.6; 0.03 M Tris (base); 0.1 mM dithiothreitol; 1 mM $CoCl_2$; 1 mM dATP; and 1 unit terminal transferase (product of BRL) at 37°C for 20 minutes to obtain about 1.5 µg of a product wherein about 100 deoxyadenosine residues were added to both 3' ends of the double-stranded DNA.

(II) Preparation of vector DNA (plasmid pBR322).

In an alternative procedure, 10 µg of *Escherichia coli* plasmid pBR322 DNA (product of BRL) was treated at 37°C for 2 hours with a restriction endonuclease EcoRI in 100 µl of a reaction mixture consisting of 10 mM Tris-HCl, pH 7.5; 6 mM $MgCl_2$; 0.1 M NaCl; 6 mM mercaptoethanol; and 10 units EcoRI (product of BRL) leading to cleavage at the unique EcoRI site in pBR322 DNA. The thus linearized plasmid DNA was treated with an exonuclease derived from phage λ in 200 µl of a reaction mixture consisting of 10 µg DNA; 0.1 M Na-glycine, pH 9.5; 5 mM $MgCl_2$; 50 µg/ml albumin (product of Merck & Co., USA); and 17.5 units λ exonuclease (product of Miles Laboratories Inc., USA) at 0°C for 90 minutes. After digestion the enzyme was removed by phenol extraction. The DNA was treated with a terminal transferase in 50 µl of a reaction mixture [10 µg DNA; 0.14 M potassium cacodylate, pH 7.6; 0.03 M Tris (base); 0.1 mM dithiothreitol; 1 mM $CoCl_2$; 1 mM dTTP; 2 units terminal transferase] at 37°C for 20 minutes to obtain about 0.5 µg of a product wherein about 100 deoxythymidine residues were added to both 3' ends of plasmid pBR322 DNA described above.

(III) Construction of recombinant DNA.

Then, 0.02 µg of the synthesized double stranded and poly(A)-tailed DNA obtained above, and 0.1 µg of the poly(T)-tailed plasmid pBR322 DNA were incubated for hybridization in 100 µl of a solution containing 0.1 M NaCl, 50 mM Tris-HCl (pH 7.5) and 5 mM EDTA at 65°C for 2 minutes, at 45°C for one hour at 37°C for one hour and at room temperature for one hour. Then, *Escherichia coli* χ1776 was transformed with the hybridized recombinant plasmid DNA following the method of Enea et al.

About 4,000 ampicillin-resistant strains were isolated by this method. 3,600 resistant strains were chosen, and the DNA of each strain was fixed on nitrocellulose filters in duplicate (Grunstein-Hogness Method).

(IV) Differential colony hybridization (synthesis of Probe A and Probe B).

In a further procedure, [32P] labelled single stranded DNA was synthesized (about 0.44 µg, specific

**0 028 033**

radioactivity approx. $6 \times 10^8$ c.p.m./µg) by reverse transcriptase in the same way as that for single stranded DNA mentioned above (see (I), step a) (dCTP was labelled with $^{32}$P) using the interferon mRNA containing fraction (about 10 µg) which had been extracted and partially purified as described above (see (I)), as a template. The DNA was hybridized in 50 µl of a reaction mixture (25 µg mRNA; 0.45 µg single stranded DNA labelled with $^{32}$P; 0.5 M NaCl; 25 mM Pipes buffer, pH 6.5) at 65°C for 40 hours with 25 µg of mRNA extracted from human fibroblasts which had not been induced by poly(I):poly(C). The latter mRNA was prepared by the same method used to extract poly(I):poly(C)-induced mRNA.

The reaction mixture was put on a column packed with 0.2 g of a hydroxyapatite, and elution was first carried out with 5 ml of 0.14 M phosphate buffer (pH 6.5) to elute the single stranded cDNA and then with 5 ml of 0.4 M phosphate buffer to elute the DNA hybridized with RNA. As a result, the DNA (about 90% of the total) (Probe A) which hybridized with the mRNA mentioned above, and the DNA (about 10% of the total) (Probe B) which did not hybridize were isolated.

Each probe was then hybridized separately with the above DNA of the ampicillin resistant transformants fixed on the nitrocellulose filters according to the Grunstein-Hogness method. Four strains were identified which reacted mainly to Probe B but little to Probe A under autoradiography.

Table 2 shows the extent of reaction of the DNAs from the four strains to each probe as revealed by autoradiogram.

TABLE 2

| Ampicillin-resistant strains, No. | Extent of reaction of Probe with DNA in the strains | |
|---|---|---|
| | Probe A | Probe B |
| No. 319 | ++ | ++++ |
| No. 644 | + | +++ |
| No. 746 | − | ++ |
| No. 3578 | + | ++++ |

(V) Selection of the strain containing the DNA complementary to the interferon mRNA.

Plasmid DNA was isolated from cells of the four strains by the method of Currier and Nester (Analyt. Biochem. vol. 76, p. 431—441, 1976). Then, these DNAs were hybridized with the interferon mRNA as follows.

First, 5 µg of plasmid DNA was linearized by incubating with restriction endonuclease Hind III, which can be obtained from Haemophilus influenzae Rd, in 50 µl of a reaction mixture consisting of 10 mM Tris-HCl, pH 7.5; 6 mM MgCl$_2$; 50 mM NaCl; 6 mM mercaptoethanol; and 5 units Hind III (product of BRL) at 37°C for 2 hours. After deproteinization by phenol extraction, the DNA was precipitated with ethanol and dissolved in 20 µl of 80% (w/v) formamide. The dissolved plasmid DNA was heated to 85°C for 10 minutes in order to separate the strands, and was then incubated in a solution consisting of 2.5 µg mRNA including interferon mRNA (see (I)), 20 µl 80% (w/v) formamide, 20 mM Pipes buffer (pH 6.5), 0.4 M NaCl and 5 mM EDTA, at 53°C. Four hours later the mixture was mixed with 0.4 ml of 3×SSC (1×SSC corresponds to 0.15 M NaCl, 0.015 M sodium citrate) at 0°C, and was filtered through a nitrocellulose filter (diameter: 1 cm, pore size: 0.45 µm) at a rate of about 0.5 ml per minute. Under these conditions only hybridized mRNA will be retained by the filter, whereas unhybridized single stranded mRNA will pass through. After washing the filter with about 1.5 ml of 2×SSC, the filter was immersed in a solution consisting of 0.6 ml of 90% (v/v) formamide, 20 mM Pipes buffer, 0.1% SDS (sodium dodecylsulfate) and 5 mM EDTA and incubated at 60°C for 2 minutes. Then the solution was removed and replaced by a new volume of this solution. Incubation of the filter and removal of the solution were repeated 3 times. By this procedure the filterbound plasmid DNA/mRNA complex was denatured and the RNA eluted into the solution (1.8 ml by combining three volumes). Then the RNA was precipitated with ethanol in the presence of 0.1 M NaCl. The polyadenylated mRNA was isolated from the RNA by using oligo(dT) cellulose column chromatography, dissolved in a mixture of 3 µl of 10 mM Tris-HCl (pH 7.5) and 88 mM NaCl and injected into the oocytes of *Xenopus laevis*. After 15 hours the interferon synthesized in the oocytes was determined (antiviral activity).

Table 3 shows the interferon mRNA activity of the mRNA which has hybridized with the plasmid DNA derived from the four bacterial strains mentioned above.

6

TABLE 3

| Bacterial strain | Interferon mRNA activity (unit/ml) |
|---|---|
| #319 | 360 |
| #644 | <10 |
| #746 | 15 |
| #3578 | <10 |
| pBR322DNA | <10 |

(VI) Hybridization tests of the DNAs from strain No. 319 and plasmid βGpBR322 DNA with rabbit β-globin mRNA and interferon mRNA.

Five µg of plasmid DNA obtained from strain No. 319 by the Currier and Nester method was cleaved with restriction endonuclease Hind III in the same manner as mentioned above. The DNA and the recombinant plasmid βGpBR322 DNA (the vector was pBR322) (obtained from the Institute for Molecular Biology I of University of Zürich or prepared by the method described in Nature *281,* 40—46, 1979) containing rabbit β-globin gene, separately or as a mixture, were hybridized with a mixture of rabbit globin mRNA (obtained from rabbit red blood cells) (1 µg) and interferon mRNA (2.5 µg) obtained from human fibroblasts under the same conditions as mentioned above (see (I)). The mRNA which formed a hybrid was injected into the oocytes of *Xenopus laevis.* The oocytes were then incubated for 15 hours in Barth's culture medium (J. Embryol. Exp. Morph. *7,* 210, 1959) containing [³H] labelled histidine, and [³H] labelled globin was isolated by acrylamide gel electrophoresis and determined quantitatively by fluorography according to the method described in Eur. J. Biochem. *46,* 83—88, (1974). The interferon was determined by antiviral activity as described above. The synthesis of rabbit β-globin and the human interferon was determined in this way. The result is shown in Table 4 below.

TABLE 4

| DNA | Synthesized interferon activity | Amount of globin synthesized |
|---|---|---|
| plasmid DNA of bacterial strain No. 319 | 200 (units/ml) | — |
| βGpBR322 | 35 | ++++ |
| mixture of both plasmids | 160 | +++ |

From the results of this experiment it has been established that plasmid DNA of strain No. 319 contains DNA which forms a hybrid specifically with the interferon mRNA.

(VII) Restriction map of the cDNA from the clone No. 319.

The plasmid DNA from clone No. 319 was cleaved with several restriction endonucleases and the resulting DNA fragments were analyzed by agarose gel electrophoresis. A restriction endonuclease cleavage map, Fig. 1(a), was prepared by standard procedures (Nucleic Acid Research, vol. 3, p. 2387—2399, 1976).

Restriction endonucleases Pst I, Bgl II and Hind III (sold by BRL, etc.) cleave No. 319 plasmid DNA at the sites illustrated in Fig. 1(a).

(VIII) Cloning of the plasmid No. 319-13

The segments obtained by cleaving No. 319 plasmid DNA with restriction endonucleases Pst I and Bgl II were isolated and purified by gel electrophoresis according to the method of Tabak & Flavell (Nucleic Acids Research, vol. 5, p. 2321—2332, 1978). The segments were labelled with ³²P by nick-translation according to the method of RIGBY, et al. (J. Mol. Biol. vol. 113, p. 237—251, 1977) and the labelled segment was used as a probe. Several strains containing a plasmid which shows complementarity to the probe were isolated from the above ampicillin-resistant strains according to the above method of Grunstein & Hogness (Proc. Nat. Acad. Sci. U.S.A., vol. 72, p. 3961—3965, 1975), namely the colony hybridization method. Plasmid DNAs were obtained from each of the strains according to the above method of Currier-Nester and the inserted portions thereof were cleaved with a restriction endonuclease such as Hind III. The cut plasmid DNA segments were compared in length and the longest plasmid DNA segment was selected. The plasmid was named No. 319-13.

(IX) Restriction map and primary structure of the plasmid No. 319-13.

The restriction endonuclease map of the plasmid is illustrated in Fig. 1(b), which demonstrates that the novel plasmid has a cDNA sequence containing the cDNA sequence of No. 319 plasmid DNA. Primary structure (base sequence) of the cDNA sequence inserted in the plasmid No. 319-13 was determined by the method of Maxam-Gilbert (Proc. Nat. Acad. Sci., U.S.A. vol. 74, p. 560—564, 1977). The primary structure of the cloned cDNA of No. 319-13 is given in Table 5 below.

TABLE 5

```
                    Start 1
                         -20                                          -10
    Hind II      Met    Thr   Asn   Lys   Cys   Leu   Leu   Gln   Ile   Ala   Leu   Leu   Leu   Cys   Phe   Ser   Thr   Thr   Ala   Leu   Ser   Met   Ser   Tyr
                                                                                                                                                    Start 2
                                                                                                                                                       1
    GTC  AAC    ATG   ACC   AAC   AAG   TGT   CTC   CTC   CAA   ATT   GCT   CTC   CTG   TTG   TGC   TTC   TCC   ACT   ACA   GCT   CTT   TCC   ATG   AGC   TAC
    CAG  TTG    TAC   TGG   TTG   TTC   ACA   GAG   GAG   GTT   TAA   CGA   GAG   GAC   AAC   ACG   AAG   AGG   TGA   TGT   CGA   GAA   AGG   TAC   TCG   ATG
                                         20                            40                                     60
```

```
                      Hinf I              10                                       20
    Asn   Leu   Leu   Gly   Phe   Leu   Gln   Arg   Ser   Ser   Asn   Phe   Gln   Cys   Gln   Lys   Leu   Leu   Trp   Gln   Leu   Asn   Gly   Arg   Leu   Glu

    AAC   TTG   CTT   GGA   TTC   CTA   CAA   AGA   AGC   AGC   AAT   TTT   CAG   TGT   CAG   AAG   CTC   CTG   TGG   CAA   TTG   AAT   GGG   AGG   CTT   GAA
    TTG   AAC   GAA   CCT   AAG   GAT   GTT   TCT   TCG   TCG   TTA   AAA   GTC   ACA   GTC   TTC   GAG   GAC   ACC   GTT   AAC   TTA   CCC   TCC   GAA   CTT
     80                            100                                 120                                   140
```

```
     30                                                         40                                    Pst I               50
    Tyr   Cys   Leu   Lys   Asp   Arg   Met   Asn   Phe   Asp   Ile   Pro   Glu   Glu   Ile   Lys   Gln   Leu   Gln   Gln   Phe   Gln   Lys   Glu   Asp   Ala

    TAT   TGC   CTC   AAG   GAC   AGG   ATG   AAC   TTT   GAC   ATC   CCT   GAG   GAG   ATT   AAG   CAG   CTG   CAG   CAG   TTC   CAG   AAG   GAG   GAC   GCC
    ATA   ACG   GAG   TTC   CTG   TCC   TAC   TTG   AAA   CTG   TAG   GGA   CTC   CTC   TAA   TTC   GTC   GAC   GTC   GTC   AAG   GTC   TTC   CTC   CTG   CGG
           160                           180                                 200                                 220
```

```
                        60                                          70                  Hinf I                          80
    Ala   Leu   Thr   Ile   Tyr   Glu   Met   Leu   Gln   Asn   Ile   Phe   Ala   Ile   Phe   Arg   Gln   Asp   Ser   Ser   Ser   Thr   Gly   Trp   Asn   Glu

    GCA   TTG   ACC   ATC   TAT   GAG   ATG   CTC   CAG   AAC   ATC   TTT   GCT   ATT   TTC   AGA   CAA   GAT   TCA   TCT   AGC   ACT   GGC   TGG   AAT   GAG
    CGT   AAC   TGG   TAG   ATA   CTC   TAC   GAG   GTC   TTG   TAG   AAA   CGA   TAA   AAG   TCT   GTT   CTA   AGT   AGA   TCG   TGA   CCG   ACC   TTA   CTC
           240                           260                                 280                                 300
```

```
                                                  90                                                  100
    Thr   Ile   Val   Glu   Asn   Leu   Leu   Ala   Asn   Val   Tyr   His   Gln   Ile   Asn   His   Leu   Lys   Thr   Val   Leu   Glu   Glu   Lys   Leu   Glu

    ACT   ATT   GTT   GAG   AAC   CTC   CTG   GCT   AAT   GTC   TAT   CAT   CAG   ATA   AAC   CAT   CTG   AAG   ACA   GTC   CTG   GAA   GAA   AAA   CTG   GAG
    TGA   TAA   CAA   CTC   TTG   GAG   GAC   CGA   TTA   CAG   ATA   GTA   GTC   TAT   TTG   GTA   GAC   TTC   TGT   CAG   GAC   CTT   CTT   TTT   GAC   CTC
                 320                                 340                                 360                                 380
```

TABLE 5 (Cont'd)

|     |     | 110 |     |     |     |     |     |     |     |     |     | 120 |     |     |     |     |     |     |     | Hinf I |     | 130 |     |     |     |
|-----|-----|-----|-----|-----|-----|-----|-----|-----|-----|-----|-----|-----|-----|-----|-----|-----|-----|-----|-----|-----|-----|-----|-----|-----|-----|
| Lys | Glu | Asp | Phe | Thr | Arg | Gly | Lys | Leu | Met | Ser | Ser | Leu | His | Leu | Lys | Arg | Tyr | Tyr | Gly | Arg | Ile | Leu | His | Tyr | Leu |
| AAA | GAA | GAT | TTC | ACC | AGG | GGA | AAA | CTC | ATG | AGC | AGT | CTG | CAC | CTG | AAA | AGA | TAT | TAT | GGG | AG·G | ATT | C·TG | CAT | TAC | CTG |
| TTT | CTT | CTA | AAG | TGG | TCC | CCT | TTT | GAG | TAC | TCG | TCA | GAC | GTG | GAC | TTT | TCT | ATA | ATA | CCC | TC·C | TAA | G·AC | GTA | ATG | GAC |
|     |     |     | 400 |     |     |     |     |     | 420 |     |     |     |     |     |     | 440 |     |     |     |     |     |     | 460 |     |     |

(Hinf I site boxed: G ATT C / C TAA G)

| Hae III |     |     |     |     |     | 140 |     |     |     |     |     |     |     |     |     | 150 |     |     |     |     |     |     |     |     |     |
|-----|-----|-----|-----|-----|-----|-----|-----|-----|-----|-----|-----|-----|-----|-----|-----|-----|-----|-----|-----|-----|-----|-----|-----|-----|-----|
| Lys | Ala | Lys | Glu | Tyr | Ser | His | Cys | Ala | Trp | Thr | Ile | Val | Arg | Val | Glu | Ile | Leu | Arg | Asn | Phe | Tyr | Phe | Ile | Asn | Arg |
| AA·G | GCC | AAG | GAG | TAC | AGT | CAC | TGT | GCC | TGG | ACC | ATA | GTC | AGA | GTG | GAA | ATC | CTA | AGG | AAC | TTT | TAC | TTC | ATT | AAC | AGA |
| TT·C | CGG | TTC | CTC | ATG | TCA | GTG | ACA | CGG | ACC | TGG | TAT | CAG | TCT | CAC | CTT | TAG | GAT | TCC | TTG | AAA | ATG | AAG | TAA | TTG | TCT |
|     |     |     | 480 |     |     |     |     |     | 500 |     |     |     |     |     |     |     | 520 |     |     |     |     |     | 540 |     |     |

(Hae III site boxed: G GCC / C CGG)

| 160 |     |     |     |     |     | 166 | Stop | Bgl II |     |     |     |     |     |     |     |     |     |     |     |     |     |     |     |     |     |
|-----|-----|-----|-----|-----|-----|-----|------|--------|-----|-----|-----|-----|-----|-----|-----|-----|-----|-----|-----|-----|-----|-----|-----|-----|-----|
| Leu | Thr | Gly | Tyr | Leu | Arg | Asn |      |        |     |     |     |     |     |     |     |     |     |     |     |     |     |     |     |     |     |
| CTT | ACA | GGT | TAC | CTC | CGA | AAC | TGA  | AGA | TCT | CCT | AGC | CTG | TGC | CTC | TGG | GAC | TGG | ACA | ATT | GCT | TCA | AGC | ATT | CTT | CAA |
| GAA | TGT | CCA | ATG | GAG | GCT | TTG | ACT  | TCT | AGA | GGA | TCG | GAC | ACG | GAG | ACC | CTG | ACC | TGT | TAA | CGA | AGT | TCG | TAA | GAA | GTT |
|     |     |     |     | 560 |     |     |      |     |     |     | 580 |     |     |     |     |     | 600 |     |     |     |     |     | 620 |     |     |

(Bgl II site boxed: AGA TCT / TCT AGA)

| CCA | GCA | GAT | GCT | GTT | TAA | GTG | ACT | GAT | GGC | TAA | TGT | ACT | GCA | TAT | GAA | AGG | ACA | CTA | GAA | GAT | TTT | GAA | ATT | TTT | ATT |
|-----|-----|-----|-----|-----|-----|-----|-----|-----|-----|-----|-----|-----|-----|-----|-----|-----|-----|-----|-----|-----|-----|-----|-----|-----|-----|
| GGT | CGT | CTA | CGA | CAA | ATT | CAC | TGA | CTA | CCG | ATT | ACA | TGA | CGT | ATA | CTT | TCC | AGT | GAT | CTT | CTA | AAA | CTT | TAA | AAA | TAA |
|     |     |     |     |     | 640 |     |     |     |     |     | 660 |     |     |     |     |     | 680 |     |     |     |     |     |     | 700 |     |

| AAA | TTA | TGA | GTT | ATT | TTT | ATT | TAT | TTA | AAT | TTT | ATT | TTG | GAA | AAT | AAA | TTA | TTT | TTG | GTG | CAA | AAG | TCA | AAA | AAA |
|-----|-----|-----|-----|-----|-----|-----|-----|-----|-----|-----|-----|-----|-----|-----|-----|-----|-----|-----|-----|-----|-----|-----|-----|-----|
| TTT | AAT | ACT | CAA | TAA | AAA | TAA | ATA | AAT | TTA | AAA | TAA | AAC | CTT | TTA | TTT | AAT | AAA | AAC | CAC | GTT | TTC | AGT | TTT | TTT |
|     |     |     |     |     | 720 |     |     |     |     |     |     | 740 |     |     |     |     |     | 760 |     |     |     |     |     |     |

0 028 033

# 0 028 033

The DNA sequence permits prediction of the entire amino acid sequence for human fibroblast interferon (amino acids 1—166) and its putative signal peptide (amino acids −21 to −1) as shown in the line above the DNA sequences.

It is important that in the sequence there exist without any errors the base triplets (three base pairs) corresponding to the amino acid sequence from the amino-terminal to the 13th amino acid of the human fibroblast interferon reported by Knight, et al. (Science vol. 207, p. 525—526, 1980). This fact is a good indication that the No. 319-13 plasmid of the present invention contains the DNA sequence coding for the human fibroblast interferon.

Further, it is apparent from the data of the primary sequence that the plasmid encompasses the entire coding region of the protein and probably the coding region of the signal peptide.

Therefore, transformation of the plasmid or cDNA inserted therein to other expression plasmids enables a host such as *Escherichia coli* to produce interferon. For such purposes, the No. 319-13 plasmid which is named TpIF 319-13, transformed in *Escherichia coli* χ1776, has been deposited with the American Type Culture Collection, Rockville, Maryland, U.S.A. under accession number ATCC 31712 and is freely available to the public.

## Claims for the Contracting States: BE, CH, DE, FR, GB, IT, LI, NL, SE

1. A double-stranded DNA which codes for a polypeptide with human fibroblast interferon activity and which is complementary to a human fibroblast interferon mRNA which directs the synthesis of biologically active human fibroblast interferon in the Xenopus laevis oocyte translation system.

2. The DNA according to claim 1, wherein the polypeptide is illustrated by the amino acid numbers 1 to 166 in Table 5.

3. The DNA according to claim 2, wherein the nucleotide sequence coding for the amino acids 1 to 166 is preceded by up to 69 nucleotides according to Table 5.

4. The DNA according to claim 2 or 3, wherein the codon for amino acid 166 is followed by up to 210 nucleotides according to Table 5.

5. The DNA according to claim 1 in cloned form.

6. The DNA according to claim 2 in cloned form.

7. The DNA according to claim 3 in cloned form.

8. A recombinant plasmid wherein a DNA according to any one of claims 1 to 7 is inserted in a vector DNA.

9. The recombinant plasmid TpIF 319-13.

10. A microorganism for cloning only containing the recombinant plasmid according to claim 8 or 9.

11. The microorganism according to claim 10 which is *Escherichia coli* χ1776.

12. *Escherichia coli* χ1776/TpIF 319-13 (=ATCC 31712).

## Claims for the Contracting State: AT

1. Process for preparing a double-stranded DNA which codes for a polypeptide with human fibroblast interferon activity and which is complementary to a human fibroblast interferon mRNA which directs the synthesis of biologically active human fibroblast interferon in the Xenopus laevis oocyte translation system, characterized in that messenger RNA including human interferon mRNA containing polyadenylic acid is used as a template.

2. Process according to claim 1, characterized in that the double-stranded DNA contains the nucleotide sequence coding for the amino acids 1 to 166 in Table 5.

3. Process according to claim 2, characterized in that the nucleotide sequence coding for the amino acids 1 to 166 is preceded by up to 69 nucleotides according to Table 5.

4. Process according to claim 2 or 3, characterized in that the codon for amino acid 166 is followed by up to 210 nucleotides according to Table 5.

5. Process according to any one of claims 1 to 4, characterized in that the DNA is inserted into a vector DNA to form a recombinant plasmid.

6. Process according to claim 5, characterized in that the recombinant plasmid is introduced into a microorganism to form a transformant.

7. Process according to claim 6, characterized in that the microorganism is a transformable *Escherichia coli* strain.

## Patentansprüche für die Vertragsstaaten: BE, CH, DE, FR, GB, IT, LI, NL, SE

1. Doppelsträngige DNA, die für ein Polypeptid mit der Aktivität menschlichen Fibroblasten-Interferons codiert und die komplementär zu einer mRNA menschlichen Fibroblasten-Interferons ist, die die Synthese biologisch aktiven menschlichen Fibroblasten-Interferons im Xenopus laevis-Oozyten-Translations-System bewirkt.

2. DNA nach Anspruch 1, wobei das Polypeptid durch die Aminosäuren Nr. 1 bis 166 in Tabelle 5 erläutert wird.

11

3. DNA nach Anspruch 2, wobei der für die Aminosäuren 1 bis 166 codierenden Nucleotid-Sequenz bis zu 69 Nucleotide gemäß Tabelle 5 vorausgehen.

4. DNA nach Anspruch 2 oder 3, wobei auf das Coden für die Aminosäure 166 bis zu 210 Nucleotide gemäß Tabelle 5 folgen.

5. DNA nach Anspruch 1 in clonierter Form.

6. DNA nach Anspruch 2 in clonierter Form.

7. DNA nach Anspruch 3 in clonierter Form.

8. Rekombinantes Plasmid, in dem eine DNA nach einem der Ansprüche 1 bis 7 in eine Vektor-DNA inseriert ist.

9. Rekombinantes Plasmid TpIF 319-13.

10. Mikroorganismus zur Clonierung, der ein rekombinantes Plasmid nach Anspruch 8 oder 9 enthält.

11. Mikroorganismus nach Anspruch 10, der *Escherichia coli* χ1776 ist.

12. *Escherichia coli* χ1776/TpIF 319-13 (=ATCC 31712).

## Patentansprüche für den Vertragsstaat: AT

1. Verfahren zur Herstellung einer doppelsträngigen DNA, die für ein Polypeptid mit der Aktivität menschlichen Fibroblasten-Interferons codiert und die komplementär zu einer menschlichen Fibroblasten-Interferon-mRNA ist, die die Synthese biologisch aktiven menschlichen Fibroblasten-Interferons im Xenopus laevis-Oozyten-Translations-System bewirkt, dadurch gekennzeichnet, daß menschliche Interferon-mRNA-enthaltende mRNA, enthaltend Polyadenylsäure, als Matrize verwendet wird.

2. Verfahren nach Anspruch 1, dadurch gekennzeichnet, daß die doppelsträngige DNA die für die Aminosäuren 1 bis 166 in Tabelle 5 codierende Nucleotidsequenz enthält.

3. Verfahren nach Anspruch 2, dadurch gekennzeichnet, daß der für die Aminosäuren 1 bis 166 codierenden Nucleotidsequenz bis zu 69 Nucleotide nach Tabelle 5 vorausgehen.

4. Verfahren nach Anspruch 2 oder 3, dadurch gekennzeichnet, daß auf das Codon für Aminosäure 166 bis zu 210 Nucleotide gemäß Tabelle 5 folgen.

5. Verfahren nach einem der Ansprüche 1 bis 4, dadurch gekennzeichnet, daß die DNA in eine Vektor-DNA unter Bildung eines rekombinanten Plasmids inseriert ist.

6. Verfahren nach Anspruch 5, dadurch gekennzeichnet, daß das rekombinante Plasmid in einen Mikroorganismus unter Bildung einer Transformante eingeführt ist.

7. Verfahren nach Anspruch 6, dadurch gekennzeichnet, daß der Mikroorganismus ein transformierbarer Stamm von *Escherichia coli* ist.

## Revendications pour les Etats Contractants: BE, CH, DE, FR, GB, IT, LI, NL, SE

1. ADN double brin qui code pour un polypeptide à activité d'interféron de fibroblastes humains et qui est complémentaire d'un mARN d'interféron de fibroblastes humains, qui dirige la synthèse de l'interféron de fibroblastes humains à activité biologique dans le système de traduction d'oocytes de Xenopus laevis.

2. ADN suivant la revendication 1, caractérisé en ce que le polypeptide est illustré par les aminoacides No. 1 à 166 du tableau 5.

3. ADN suivant la revendication 2, caractérisé en ce que la séquence des nucléotides codant pour les aminoacides 1 à 166 est précédée par jusqu'à 69 nucléotides suivant le tableau 5.

4. ADN suivant la revendication 2 ou 3, caractérisé en ce que le codon pour l'aminoacide 166 est suivi par jusqu'à 210 nucléotides suivant le tableau 5.

5. ADN suivant la revendication 1, sous forme clonée.

6. ADN suivant la revendication 2, sous forme clonée.

7. ADN suivant la revendication 3, sous forme clonée.

8. Plasmide recombinant, dans lequel un ADN suivant l'une quelconque des revendications 1 à 7 est inséré dans un ADN vecteur.

9. Plasmide recombinant TpIF 319-13.

10. Microorganisme pour cloner uniquement, contenant le plasmide recombinant suivant la revendication 8 ou 9.

11. Microorganisme suivant la revendication 10, caractérisé en ce qu'il est *Escherichia coli* χ1776.

12. *Escherichia coli* χ1776/TpIF 319-13 (=ATCC 31712).

## Revendications pour l'Etat Contractant: AT

1. Procédé de préparation d'un ADN double brin qui code pour un polypeptide à activité d'interféron de fibroblastes humains et qui est complémentaire d'un mARN d'interféron de fibroblastes humains, qui dirige la synthèse de l'interféron de fibroblastes humains à activité biologique dans le système de traduction d'oocytes de Xenopus laevis, caractérisé en ce que on utilise un ARN messager y compris du mARN d'interféron humain contenant de l'acide polyadénylique, à titre de gabarit.

2. Procédé suivant la revendication 1, caractérisé en ce que l'ADN à double brin contient la séquence des nucléotides codant pour les aminoacides 1 à 66 présentés dans le tableau 5.

**0 028 033**

3. Procédé suivant la revendication 2, caractérisé en ce que la séquence de nucléotides codant pour les aminoacides 1 à 166 est précédée de jusqu'à 69 nucleotides suivant le tableau 5.

4. Procédé suivant la revendication 2 ou 3, caractérisé en ce que le codon pour l'aminoacide 166 est suivi de jusqu'à 210 nucléotides suivant le tableau 5.

5. Procédé suivant l'une quelconque des revendications 1 à 4, caractérisé en ce que l'ADN est inséré dans un ADN vecteur pour former un plasmide recombinant.

6. Procédé suivant la revendication 5, caractérisé en ce que le plasmide recombinant est introduit dans un microorganisme pour former un transformant.

7. Procédé suivant la revendication 6, caractérisé en ce que le microorganisme est une souche d'*Escherichia coli* transformable.

Fig. 1

(a)  # 319

PstI       BglⅡ    Hind Ⅲ

(b)  # 319 −13

HincⅡ     PstI       HaeⅢ   BglⅡ      HindⅠ

Hinf I     Hinf I     HinfI